# EUROPEAN PATENT APPLICATION

(11) **EP 3 875 090 A1**
(43) Date of publication of application: **08.09.2021**
(21) Application number: 19880659.8
(22) Date of filing: 31.10.2019
(51) Int. Cl.: A61K 31/505, A61K 9/14, A61K 47/12, A61K 47/44, A61P 35/02

(54) **GRANULE CONTAINING ANTI-TUMOR AGENT**

(30) Priority: 31.10.2018 JP 2018204849
(71) Applicant: FUJIFILM Corporation, Tokyo 106-8620 (JP)
(72) Inventor: OURA Tai, Toyama-shi, Toyama 930-8508 (JP)
(74) Representative: Hoffmann Eitle
(86) International application number: PCT/JP2019/042748
(87) International publication number: WO 2020/090968

(57) **Abstract**

An object of the present invention is to provide a granulated product containing a succinate salt of (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide (hereinafter, referred to as Compound A), which has excellent stability. According to the present invention, there is provided a granulated product containing a succinate salt of (S,E)-N-(1-((5-(2-((4-cyanophenyl) amino)-4-(propylamino)pyrimidin-5-yl)-4-pentyn-1-yl)ami no)-1-oxopropan-2-yl)-4-(dimethylamino)-N-methyl-2-butenamide and at least one or more additives of the group consisting of magnesium stearate, sodium stearyl fumarate, and hydrogenated oil.

## Description

### Field of the Invention

The present invention relates to a granulated product containing a succinate salt of (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide (hereinafter, referred to as Compound A), which is a granulated product having improved stability.

### Description of the Related Art

Compound A is a medically useful compound that has an FLT3 inhibitory action and has a strong effect on hematological cancer such as acute myeloid leukemia (Patent Document 1). In the medical field, there is a demand on a highly effective and stable pharmaceutical drug, which is expected to be applied clinically in a wide range of administration formulation forms such as an oral agent, an injection agent, an ointment, and a suppository.

On the other hand, with the advancement of in-home medical care and the promotion of self-medication, the maintenance of medication adherence is an important issue in drug therapy. The most commonly used formulation form in-home medical care is an oral agent (Non-Patent Document 1).

### Prior Art Documents

### Patent Documents

### Patent Document 1: WO2015/056683A

### Non-Patent Documents

Non-Patent Document 1: Japanese Journal of Pharmaceutical Health Care and Sciences, Vol. 34, No. 3, pp. 289 to 296, 2008

### SUMMARY OF THE INVENTION

In a granulated product containing a succinate salt of Compound A, which is produced by a general granulation method, the amounts of related substances significantly increase over time as compared with the succinate salt of Compound A, and thus the stability of the succinate thereof is significantly reduced. Producing a stable granulated powder having high fluidity in the granulation process is important for improving productivity and for ensuring content uniformity. There is a strong demand on a stable granulated product of the succinate salt of compound A.

Under these circumstances, as a result of intensive studies, the inventors of the present invention have found that an increase in related substances can be suppressed in a case where one or more additives selected from magnesium stearate, sodium stearyl fumarate, and hydrogenated oil are blended. The present invention provides a stable granulated product containing a succinate salt of compound A.

The present invention provides the followings.
<1> A granulated product comprising a succinate salt of (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide and at least one or more additives selected from the group consisting of magnesium stearate, sodium stearyl fumarate, and hydrogenated oil.
<2> The granulated product according to <1>, in which the at least one or more additives selected from the group consisting of magnesium stearate, sodium stearyl fumarate, and hydrogenated oil are magnesium stearate.
<3> The granulated product according to <1>, in which the at least one or more additives selected from the group consisting of magnesium stearate, sodium stearyl fumarate, and hydrogenated oil is sodium stearyl fumarate.
<4> The granulated product according to <1>, in which the at least one or more additives selected from the group consisting of magnesium stearate, sodium stearyl fumarate, and hydrogenated oil are hydrogenated oil.

The granulated product containing the succinate salt of compound A according to an aspect of the present invention is a granulated product having excellent stability, in which the increase in related substances is suppressed.

### DESCRIPTION OF THE PREFERRED EMBODIMENTS

The present invention will be described in detail below.

As used in the present specification, % means the mass percentage unless otherwise particularly specified. In the present invention, the numerical range indicated by using "to" indicates a range including numerical values described before and after "to" as a minimum value and a maximum value, respectively. In addition, in the present invention, in a case where there are a plurality of substances corresponding to each component in the composition, the amount of each component in the composition means the total amount of the plurality of substances present in the composition unless otherwise particularly specified.

A succinate salt of Compound A used in the present invention can be produced, for example, by the method disclosed in WO2015/056683A.

Examples of magnesium stearate which is used in the present invention include Parteck™ LUB MST (Merck KGaA), magnesium stearate (vegetable) (Taihei Chemical Industrial Co., Ltd.), and Japanese Pharmacopeia Magnesium stearate JPM (Sakai Chemical Industry Co., Ltd.). Examples of sodium stearyl fumarate include PRUV (JRS Pharma). Examples of the hydrogenated oil include Lubriwax 101 (Freund Corporation) and Lubriwax 103 (Freund Corporation).

The content of magnesium stearate, sodium stearyl fumarate, or hydrogenated oil, which is used in the present invention, is 0.1% to 35%, preferably 0.2% to 15%, and more preferably 0.5% to 10%, with respect to the total amount.

An excipient may be added to the granulated product according to the embodiment of the present invention, as necessary.

Examples of the excipient include sugar alcohols such as erythritol, mannitol, xylitol, lactitol, isomalt, and sorbitol; sugars such as sucrose, lactose, maltose, hydrated trehalose, and glucose; cyclodextrins such as α-cyclodextrin, β-cyclodextrin, γ-cyclodextrin, hydroxypropyl β-cyclodextrin, and sulfobutyl ether β-cyclodextrin sodium; celluloses such as microcrystalline cellulose; and starches such as corn starch, potato starch, and pregelatinized starch. These excipients may be added alone or in a combination of two or more thereof.

Examples of the preferred excipient include sugars, sugar alcohols, and celluloses.

The sugars are more preferably lactose, and the sugar alcohols are more preferably mannitol or erythritol and still more preferably erythritol. The celluloses are preferably microcrystalline cellulose.

The amount of the excipient added is not particularly limited, and an amount determined depending on the dosage form may be added.

In the granulated product according to the embodiment of the present invention, a pharmaceutically acceptable pharmaceutical aid can be used within the range in which the effects of the present invention are not impaired.

Examples of the pharmaceutical aid include a disintegrant, a binder, a sweeting agent, a coloring agent, a flavoring agent, a surfactant, a coating agent, and a plasticizer.

Examples of the disintegrant include carmellose, carmellose calcium, croscarmellose sodium, carboxymethyl starch sodium, crospovidone, low-substituted hydroxypropylcellulose, and partially pregelatinized starch.

Examples of the binder include hydroxypropyl cellulose, carmellose sodium, and methyl cellulose, hypromellose, and polyvinyl alcohol.

Examples of the sweeting agent include aspartame, saccharin, stevia, thaumatin, and acesulfame potassium.

Examples of the colorant include titanium dioxide, red ferric oxide, yellow ferric oxide, black iron oxide, Food Red No. 102, Food Yellow No. 4, and Food Yellow No. 5.

Examples of the flavoring agent include essential oils such as orange oil, lemon oil, peppermint oil, and pine oil; extracts such as orange extract and peppermint extract; flavors such as cherry flavor, vanilla flavor, and fruit flavor; powdered fragrances such as apple micron, banana micron, peach micron, strawberry micron, and orange micron; vanillin; and ethyl vanillin.

Examples of the surfactant include sodium lauryl sulfate, sodium dioctyl sulfosuccinate, polysorbate, and polyoxyethylene-hardened castor oil.

Examples of the coating agent include hypromellose, an aminoalkyl methacrylate copolymer E, an aminoalkyl methacrylate copolymer RS, ethyl cellulose, cellulose acetate phthalate, hydroxypropyl methyl cellulose phthalate, a methacrylic acid copolymer L, a methacrylic acid copolymer LD, and a methacrylic acid copolymer S.

Examples of the plasticizer include triethyl citrate, macrogol, triacetin, and propylene glycol.

These pharmaceutical aids may be used alone or in a combination of two or more thereof.

The blending amount of the pharmaceutical aid is not particularly limited, and blending may be carried out appropriately so that the effect thereof is sufficiently exhibited according to each purpose.

The granulated product according to the embodiment of the present invention is obtained by mixing a succinate salt of Compound A with one or more additives selected from the group consisting of magnesium stearate or sodium stearyl fumarate and hydrogenated oil to prepare a mixed powder, and granulating the mixed powder.

The granulated product according to the embodiment of the present invention is obtained by, for example, mixing a succinate salt of Compound A with one or more additives selected from the group consisting of magnesium stearate or sodium stearyl fumarate and hydrogenated oil, and further with an excipient and/or a pharmaceutical aid to produce a mixed powder, compressing the obtained mixed powder by a dry-type granulation method to produce a compression-molded product, and crushing and sizing the obtained compression-molded product.

The granulation method is not particularly limited, and examples thereof include a wet-type granulation method, a dry-type granulation method, and a melting granulation method, preferred examples thereof include a wet-type granulation method and a dry-type granulation method, and more preferred examples thereof include a dry-type granulation method.

Examples of the wet-type granulation method include a fluidized bed type granulation method, a wet-type crushing granulation method, a rotary granulation method, a spray-drying type granulation method, and an extrusion granulation method, preferred examples thereof include a fluidized bed type granulation method and the wet-type crushing granulation method, and more preferred examples thereof include a fluidized bed type granulation method.

Examples of the dry-type granulation method include a compacting method, a slugging method, and a briquetting method, and preferred examples thereof include a compacting method.

Examples of the compacting method include a method of using a roller compactor. In a case where a roller compactor is used, the pressure at the time of production is preferably 3 to 12 MPa, for example, in a case where TF-LABO (manufactured by Freund Corporation) is used.

Further, the granulated product according to the embodiment of the present invention can be made into a formulation such as a tablet, a hard capsule, granules, fine granules, powders, a fast-disintegrating tablet, a formulation dissoluble at use, a dry syrup, or a powder formulation, by appropriately using an excipient, a pharmaceutical aid, and the like which are pharmaceutically acceptable.

Examples of the preferred formulation include a hard capsule and a tablet, and more preferred examples thereof include a hard capsule.

In the process of producing the mixed powder, the granulated product according to the embodiment of the present invention can be made into an uncoated tablet, for example, by producing a mixed powder to which an excipient and a pharmaceutical aid are added, and tableting the obtained mixed powder.

Furthermore, the obtained uncoated tablet can be film-coated.

In the process of producing the mixed powder, the granulated product according to the embodiment of the present invention can be made into a hard capsule, for example, by producing a mixed powder to which an excipient and a pharmaceutical aid are added, and filling a capsule with the obtained mixed powder.

Examples of the hard capsule include capsules manufactured using gelatin, hypromellose, pullulan, and the like, and preferred examples thereof include a capsule manufactured using hypromellose.

Examples of the hard capsule include Vcaps Plus (Lonza Group AG) and Quali-V (Qualicaps Co., Ltd.). The size of the hard capsule agent is preferably No. 0 to No. 4 and more preferably No. 2 to No. 4.

In a case of being stored at 60°C for one month, the granulated product according to the embodiment of the present invention is a granulated product in which the produced amounts of related substances are preferably 1.2% or less, more preferably 0.8% or less, and still more preferably 0.6% or less.

The measurement method for related substances may be carried out by a conventional method, and examples thereof include the following method with high performance liquid chromatography (hereinafter, referred to as HPLC).

### (1) Preparation of standard solution

About 25 mg of a succinate salt of Compound A is precisely weighed, added to a dissolving solvent prepared by mixing water, acetonitrile, and trifluoroacetic acid at a ratio of 800:200:1, and irradiated with ultrasonic waves for dissolution, and then the volume is exactly adjusted to 50 mL. 1 mL of this solution is exactly quantitated and added to the dissolving solvent, and the volume is precisely adjusted to 100 mL.

### (2) Preparation of sample solution

A sample corresponding to about 25 mg of a succinate salt of Compound A is precisely weighed, added to a dissolving solvent, irradiated with ultrasonic waves for dissolution, and then the volume is exactly adjusted to 50 mL (0.5 mg/ml).

### (3) Measurement

5 µL of the standard solution and 5 µL of a sample solution are precisely quantitated and injected into an HPLC apparatus, and measurement is carried out under the following conditions.

### <Measurement condition>

Detector: ultraviolet absorption spectrophotometer (measurement wavelength: 300 nm)
Column: Meteoric Core C18, 3.0 mmcp x 150 mm, particle size: 2.7 µm
Column temperature: 40°C
Sample temperature: constant temperature around 5°C
Mobile phase A: water / trifluoroacetic acid = 1,000/1
Mobile phase B: acetonitrile / trifluoroacetic acid = 1,000/1

Feeding of mobile phase: the mixing ratio between the mobile phase A and the mobile phase B is changed as follows to control the concentration gradient.

**[Table 1]**

| Time after injection (minutes) | Mobile phase A (vol%) | Mobile phase B (vol%) |
|---|---|---|
| 0∼5 | 80 | 20 |
| 5∼65 | 80→765 | 20→35 |
| 65∼80 | 65→735 | 35→65 |

Flow rate: 0.45 mL/min

### <Calculation expression>

Amount (%) of each of related substances Qi = (WS/WT) x (Ai/AS) x 1/100 x 100 WS: weighed amount (mg) of succinate salt of Compound A
WT: weighed amount (mg) of sample
Ai: peak surface area of each of related substances in sample solution
AS: average peak surface area of standard solution
1/100: dilution correction coefficient

The concentration of the sample solution (0.5 mg/mL) is a concentration within the range in which linearity is ensured in the calibration curve of the solution concentration versus the area percentage in HPLC, and the amount (%) of each of related substances indicates a value equivalent to the area percentage of each of the related substances in the HPLC measurement. Therefore, the area of each peak is measured by the automatic integration method, and the amounts of related substances can be calculated by the area percentage method.

For example, only a sample solution of a related substance is prepared and injected into an HPLC apparatus, the area percentage of the related substance (retention time: 72 minutes) is measured by the automatic integration method, and the mass of the related substance is calculated by the area percentage method, and a value equivalent to the mass (%) of the related substance can be obtained.

### Examples

The usefulness of the present invention will be described in Examples and Comparative Examples; however, the present invention is not limited thereto.

In Examples, as a desktop V-type mixer, VM-2 manufactured by TSUTSUI Scientific Instruments Co., Ltd. was used;
as a dry-type granulator, TF-LABO manufactured by Freund Corporation was used;
as sodium stearyl fumarate, PRUV manufactured by JRS Pharma was used;
as magnesium stearate, Parteck™ LUB MST manufactured by Merck KGaA was used; and
as a hard capsule, Vcaps Plus made by Lonza Group AG was used.

### Example 1

29.7 g of a succinate salt of Compound A and 0.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product.

### Example 2

80 g of a succinate salt of Compound A and 2.6 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. A No. 1 hard capsule was filled with 94.5 mg of the obtained granulated product to obtain a hard capsule.

### Example 3

30 g of a succinate salt of Compound A and 3.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. A No. 1 hard capsule was filled with 101.7 mg of the obtained granulated product to obtain a hard capsule.

### Example 4

30 g of a succinate salt of Compound A and 0.93 g of sodium stearyl fumarate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. A No. 1 hard capsule was filled with 94.3 mg of the obtained granulated product to obtain a hard capsule.

### Example 5

30 g of a succinate salt of Compound A and 1.6 g of sodium stearyl fumarate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Sodium stearyl fumarate was added to the obtained granulated product so that the content thereof was 1% of the total amount, and the resultant mixture was mixed with the desktop V-type mixer for 30 minutes. A No. 4 hard capsule was filled with 97.3 mg of the obtained mixed powder to obtain a hard capsule.

### Example 6

30 g of a succinate salt of Compound A and 3.3 g of sodium stearyl fumarate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Sodium stearyl fumarate was added to the obtained granulated product so that the content thereof was 1% of the total amount, and the resultant mixture was mixed with the desktop V-type mixer for 30 minutes. A No. 4 hard capsule was filled with 102.7 mg of the obtained mixed powder to obtain a hard capsule.

### Example 7

30 g of a succinate salt of Compound A and 3.3 g of hydrogenated oil (Lubriwax 103, manufactured by Freund Corporation) were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. A No. 1 hard capsule was filled with 101.7 mg of the obtained granulated product to obtain a hard capsule.

### Example 8

30 g of a succinate salt of Compound A, 0.66 g of hydrogenated oil (Lubriwax 103, manufactured by Freund Corporation), and 0.98 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. A No. 1 hard capsule was filled with 96.5 mg of the obtained granulated product to obtain a hard capsule.

### Example 9

15 g of a succinate salt of Compound A, 16.4 g of erythritol (Erythritol T fine powder, manufactured by Mitsubishi-Chemical Foods Corporation), and 0.32 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.2% of the total amount, and the resultant mixture was mixed with the desktop V-type mixer for 30 minutes. A No. 3 hard capsule was filled with 193.8 mg of the obtained mixed powder to obtain a hard capsule.

### Example 10

15 g of a succinate salt of Compound A, 8.2 g of microcrystalline cellulose (KG1000, manufactured by Asahi Kasei Corporation), and 0.46 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.2% of the total amount, and the resultant mixture was mixed with the desktop V-type mixer for 30 minutes. A No. 4 hard capsule was filled with 144.6 mg of the obtained mixed powder to obtain a hard capsule.

### Comparative Example 1

30 g of a succinate salt of Compound A was sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. A No. 4 hard capsule was filled with 91.5 mg of the obtained granulated product to obtain a hard capsule.

### Comparative Example 2

30 g of a succinate salt of Compound A and 0.93 g of calcium stearate (manufactured by Taihei Chemical Industrial Co., Ltd.) were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. A No. 4 hard capsule was filled with 94.3 mg of the obtained granulated product to obtain a hard capsule.

### Comparative Example 3

30 g of a succinate salt of Compound A and 0.93 g of talc (CROWN TALC PP, manufactured by Matsumura Sangyo Co., Ltd.) were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. A No. 4 hard capsule was filled with 94.3 mg of the obtained granulated product to obtain a hard capsule.

### Test example 1

92.4 mg of the sized granulated product obtained in Example 1 and each of the capsules obtained in Examples 2 to 8 and Comparative Examples 1 to 3 were sealed in a glass bottle and stored at 60°C for 1 month. The amount of the related substance (retention time: 72 minutes) after storage was measured by HPLC. The HPLC measurement was carried out under the following conditions. The peak surface area was measured by the automatic integration method, and the amount of the related substance was calculated by the area percentage method.

### (Measurement condition)

Detector: ultraviolet absorption spectrophotometer (measurement wavelength: 300 nm)
Column: Meteoric Core C18, 3.0 mmcp x 150 mm, particle size: 2.7 µm
Column temperature: 40°C
Mobile phase A: water / trifluoroacetic acid = 1,000/1
Mobile phase B: acetonitrile / trifluoroacetic acid = 1,000/1

Mixing ratio between mobile phase during feeding: the mixing ratio between the mobile phase A to the mobile phase B was set as shown in Table 2. The concentration gradient was set to a linear gradient.

**[Table 2]**

| Time after injection (minutes) | Mobile phase A (vol%) | Mobile phase B (vol%) |
|---|---|---|
| 0∼5 | 80 | 20 |
| 5∼65 | 80→65 | 20→35 |
| 65∼80 | 65→35 | 35→65 |

Injection volume: 5 µL (a sample solution prepared to be 0.5 mg/mL was injected)
Flow rate: 0.45 mL/min

The results are shown in Table 3 and Table 4.

**[Table 3]**

| Unit (mg) | Example 1 | Example 2 | Example 3 | Example 4 | Example 5 | Example 6 |
|---|---|---|---|---|---|---|
| Succinate of Compound A | 91.5 | 91.5 | 91.5 | 91.5 | 91.5 | 91.5 |
| Magnesium stearate | 0.9 | 3.0 | 10.2 | 0.0 | 0.0 | 0.0 |
| Sodium stearyl fumarate | 0.0 | 0.0 | 0.0 | 2.8 | 5.8 | 11.2 |
| Hydrogenated oil | 0.0 | 0.0 | 0.0 | 0.0 | 0.0 | 0.0 |
| Calcium stearate | 0.0 | 0.0 | 0.0 | 0.0 | 0.0 | 0.0 |
| Talc | 0.0 | 0.0 | 0.0 | 0.0 | 0.0 | 0.0 |
| Total | 92.4 | 94.5 | 101.7 | 94.3 | 97.3 | 102.7 |
| Produced amount of related substance (%) | 0.8 | 0.7 | 0.5 | 0.7 | 0.6 | 0.7 |

**[Table 4]**

| Unit (mg) | Example 7 | Example 8 | Comparative Example 1 | Comparative Example 2 | Comparative Example 3 |
|---|---|---|---|---|---|
| Succinate of Compound A | 91.5 | 91.5 | 91.5 | 91.5 | 91.5 |
| Magnesium stearate | 0.0 | 3.0 | 0.0 | 0.0 | 0.0 |
| Sodium stearyl fumarate | 0.0 | 0.0 | 0.0 | 0.0 | 0.0 |
| Hydrogenated oil | 10.2 | 2.0 | 0.0 | 0.0 | 0.0 |
| Calcium stearate | 0.0 | 0.0 | 0.0 | 2.8 | 0.0 |
| Talc | 0.0 | 0.0 | 0.0 | 0.0 | 2.8 |
| Total | 101.7 | 96.5 | 91.5 | 94.3 | 94.3 |
| Produced amount of | 1.0 | 0.8 | 4.3 | 2.1 | 1.8 |
| related substance (%) | | | | | |

In the granulated product containing the succinate salt of Compound A, and magnesium stearate, sodium stearyl fumarate, or hydrogenated oil, the increase in related substances at 60°C for 1 month was significantly suppressed as compared with Comparative Examples.

The granulated product containing the succinate salt of Compound A according to the embodiment of the present invention has high stability and is useful.

## Claims

1. A granulated product comprising:
a succinate salt of (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide; and
at least one or more additives selected from the group consisting of magnesium stearate, sodium stearyl fumarate, and hydrogenated oil.

2. The granulated product according to claim 1, wherein the at least one or more additives selected from the group consisting of magnesium stearate, sodium stearyl fumarate, and hydrogenated oil are magnesium stearate.

3. The granulated product according to claim 1, wherein the at least one or more additives selected from the group consisting of magnesium stearate, sodium stearyl fumarate, and hydrogenated oil are sodium stearyl fumarate.

4. The granulated product according to claim 1, wherein the at least one or more additives selected from the group consisting of magnesium stearate, sodium stearyl fumarate, and hydrogenated oil are hydrogenated oil.
